# EUROPEAN PATENT APPLICATION

(11) **EP 1 555 315 A1**
(43) Date of publication of application: **20.07.2005**
(21) Application number: 03808890.2
(22) Date of filing: 22.09.2003
(51) Int. Cl.: C12N 9/98, C12N 15/09, C12P 21/02

(54) **PROTEIN SYNTHESIS SYSTEM USING XENOPUS OOCYTE EXTRACT**

(30) Priority: 18.10.2002 JP 2002303838
(71) Applicant: Riken, Wako-shi, Saitama 351-0198 (JP)
(72) Inventor: SHIROUZU, Mikako c/o RIKEN Yokohama Institute, Yokohama-shi, Kanagawa 230-0045 (JP); TOKMAKOV, Alexander c/o RIKEN Yokohama Institute, Yokohama-shi, Kanagawa 230-0045 (JP); YOKOYAMA, Shigeyuki c/o RIKEN Yokohama Institute, Yokohama-shi, Kanagawa 230-0045 (JP)
(74) Representative: Arends, William Gerrit
(86) International application number: PCT/JP2003/012059
(87) International publication number: WO 2004/035772

(57) **Abstract**

It is intended to provide a cell-free protein synthesis system with the use of a *Xenopus* oocyte extract, which is superior in protein synthesis efficiency to the microinjection method into oocytes or the existing method with the use of an egg extract. Namely, a kit for use in a cell-free protein synthesis comprising an extract prepared from *Xenopus* oocytes at the early stage of maturation entered from the state arrested at the first meiotic prophase in the cell cycle, an energy regenerating system, and at least one amino acid.

## Description

### Technical Field

The present invention relates to an oocyte extract of amphibian such as *Xenopus laevis*, in particular, an extract of oocytes at the specific stage of maturation wherein the protein synthetic activity of the oocyte is remarkably enhanced, and to a method for producing a protein in cell-free protein synthesis system using the extract.

### Background Art

*Xenopus* oocytes and eggs are widely used to study DNA replication, transcription, processing of transcripts, translation, and post-translational protein modifications. They are also used as an expression system for injected exogenous genes. In 1977, Gurdon and Brown started injecting exogenous genes into *Xenopus* oocytes and eggs to investigate their functions, and reported the method for the first time. This method is carried out by injecting mRNA into *Xenopus* oocytes or eggs, or injecting cDNA into nuclei of *Xenopus* oocytes. Previously, in the method of injecting genes such as mRNA and cDNA into *Xenopus* oocytes or eggs, a manual or automatic microinjection has been made with a pipette filled with these samples and a manipulator under microscopic observation (see, for example, Patent reference 1).

On the other hand, cell-free protein synthesis systems comprised of a variety of cell extracts containing required enzymes and the like, and energy sources have been developed. These systems provide important experimental methods for analyzing protein structure and function. The cell extracts from, such as rabbit reticulocyte, *E. coli*, wheat germ, HeLa cell, thermophilic bacteria, mouse L-cell, Ehrlich's ascitic cancer cell, CHO cell, and budding yeast and the like, are used, and several kits for cell-free protein synthesis system derived from rabbit reticulocyte, *E. coli*, and wheat germ are commercially available. Comparing the above two methods briefly, it is generally believed that the protein synthesis in cell-free system can be carried out more rapidly, and is controllable more easily, than the synthesis in *Xenopus* oocytes.

*Xenopus* oocytes are generally arrested in the cell cycle at the first meiotic prophase. Only a few number of the cells mature periodically under the influence of hormones, and complete their first meiotic division to be secondary oocytes, which are arrested at the metaphase II. The process of maturation is triggered by progesterone. This hormone is secreted by follicle cells surrounding the oocytes in the ovary, which in turn are stimulated by gonadotropins released from the pituitary. Maturation can also be induced *in vitro* by treating isolated oocytes with progesterone. During the maturation of *Xenopus* oocytes, the rate of endogenous protein synthesis is reported to show a twofold increase (see, for example, non-patent reference 1). The extract of *Xenopus* eggs that matured to metaphase II to be capable of being fertilized, can perform the post-translational modification of protein, i.e. addition of carbohydrate chain, phosphorylation, cleavage of signal peptide, localization, and formation of higher structure (see, for example, non-patent reference 2).

Higher eukaryotic mRNA can be translated in the extract of the above *Xenopus* eggs, but the translational efficiency of prokaryotic mRNA is lower than that of eukaryote. Several µg/ml levels of proteins are synthesized in a ribonuclease-treated egg extract from added synthetic mRNAs (see non-patent reference 2), however, in the oocyte extract, efficient expression of exogenous (globin) mRNA required the presence of reticulocyte postribosomal supernatant S-100, suggesting that one or more factors required for mRNA recruitment is limiting in these extracts (see non-patent reference 1).

### [Patent reference 1] Patent publication of JP-A-2002-65240

[Non-patent reference 1] Tina D. Patrick et al., Preparation and characterization of cell-free protein synthesis system from oocytes and eggs of *Xenopus laevis*, Development, vol. 106, pp.1-9, (1989)

[Non-patent reference 2] Glenn Mathhews and Alan Colman, A highly efficient, cell-free translation/translocation system prepared from *Xenopus* eggs, Nucleic Acids Research, vol. 19, No.23, pp.6405-6412, (1991)

### Disclosure of the Invention

Although the expression of exogenous genes microinjected into *Xenopus* oocytes, eggs, and early embryos has been investigated, the comparative analysis of expression of exogenous genes in extracts obtained from the cells on various stages of cell cycle has not been performed in detail. Therefore, it is an object of the present invention to provide a novel cell extract that can be used for cell-free protein synthesis system by optimizing the system using *Xenopus* oocyte extracts. It is also an object of the present invention to provide a cell-free protein synthesis system using a *Xenopus* oocyte extract, which method is superior to that of microinjection to oocytes or that of using a conventional egg extract in respect of protein synthetic efficiency.

The present invention has been completed to solve the above problems, and is based on the findings that the cell extract prepared at the specific stage of oocyte maturation shows the remarkably enhanced activity of protein synthesis and synthesizes a large amount of protein compared to the conventional method with a matured egg extract or microinjection to oocytes, as a result of investigating the relationship between the respective stages of *Xenopus* oocyte, egg (matured egg), and embryo in the cell cycle, and the efficiencies of protein expression of introduced exogenous genes in detail.

Accordingly, in a first aspect of the present invention, there is provided an extract of *Xenopus* oocytes, which extract is prepared from the oocytes at the early stage of maturation entered from the state arrested at the first meiotic prophase in the cell cycle.

In another aspect of the present invention, there is provided a kit for use in a cell-free protein synthesis comprising an extract prepared from *Xenopus* oocytes at the early stage of maturation entered from the state arrested at the first meiotic prophase in the cell cycle, an energy regenerating system, and at least one amino acid. In a preferred embodiment, the kit of the present invention further comprises a plasmid DNA for the preparation of exogenous polynucleotide.

In a still another aspect of the present invention, there is provided a method of producing a protein in a cell-free protein synthesis system comprising the steps of preparing a polynucleotide coding for a protein, and expressing said polynucleotide in an extract prepared from *Xenopus* oocytes at the early stage of maturation entered from the state arrested at the first meiotic prophase in the cell cycle.

In a preferred embodiment, the oocytes are at entry to an early stage of maturation by treating oocytes arrested at the first meiotic prophase with an egg maturation inducer. The egg maturation inducer is at least one selected from the group consisting of glucocorticoid, mineralcorticoid, progesterone, testosterone, and insulin. The polynucleotide is an exogenous mRNA that is synthesized and modified *in vitro*.

### Brief Description of the Drawings

Fig.1 is a schematic diagram that represents stages in the cell cycle of *Xenopus* oocyte, egg, and early embryo.
Fig.2 shows expression levels of Xyk in metaphase and interphase of *Xenopus* egg measured by immunoblotting.
Fig.3 shows expression levels of Xyk in *Xenopus* oocytes, an oocyte extract, and an egg extract measured by immunoblotting.
Fig.4 shows effect of progesterone on Xyk expression in microinjected oocytes analyzed by immunoblotting using immunoprecipitated samples with mAb327.
Fig.5 shows effect of progesterone on Xyk expression in microinjected oocytes measured by protein kinase activity with poly(Gly,Tyr) as a substrate.
Fig.6 shows effect of progesterone on Xyk expression in *Xenopus* oocyte extract analyzed by immunoblotting using immunoprecipitated samples with mAb327.
Fig.7 shows effect of progesterone on Xyk expression in *Xenopus* oocyte extract measured by protein kinase activity with cdc2 and Xyk (auto: expression product itself) as substrates and by the immunoprecipitation with anti-pepY antibody.
Fig.8 is a result of synthetic rate of Xyk in microinjected *Xenopus* oocytes.
Fig.9 is a result of synthetic rate of Xyk in *Xenopus* egg extracts.

### [Explanation of marks]

a.u.: arbitrary unit

### Best Mode for Carrying Out the Invention

Preferred embodiments of the present invention are explained as follows by reference to the figures.

### (Preparation method of oocyte extracts)

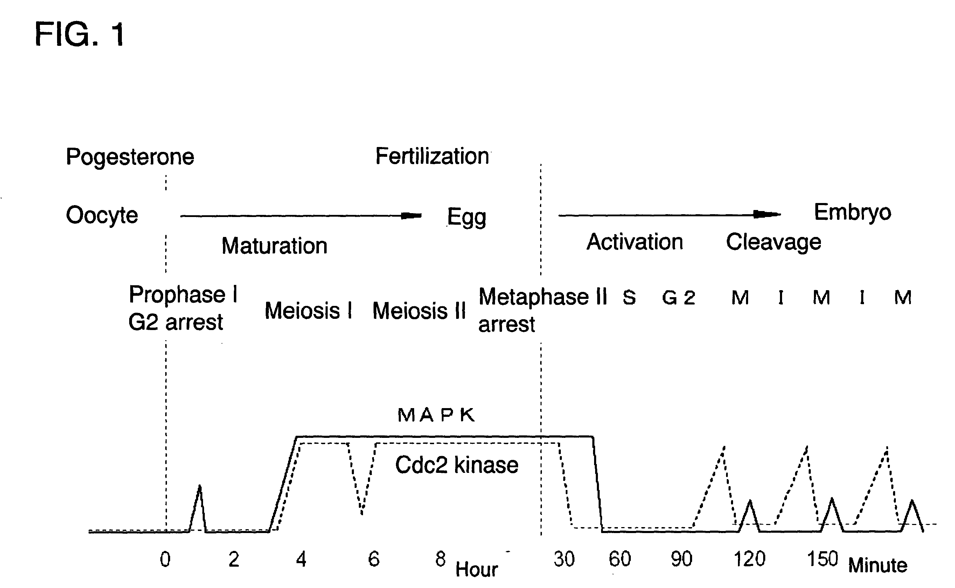

*Xenopus* oocytes, eggs and embryos pass through the different stages of cell cycle that are characterized by the distinct levels of metabolic and protein synthetic activities as shown in Fig.1, and result in maturation. Fully grown *Xenopus* oocytes are naturally arrested in the first meiotic prophase at the G2/M boundary. The oocytes have the intact nuclear envelope, partially decondensed chromatin, high activity of transcription, and low activity of the mitotic regulators (factors present in cytoplasm, which regulate the cell cycle) such as maturation promoting factor (MPF) and cytostatic factor (CSF). Immature oocytes are not competent to fertilization and can be arrested in this state for a long time until the steroid hormone progesterone, secreted from surrounding follicle cells, induces their transition from prophase I to metaphase II arrested in the process of meiotic maturation. The metaphase II arrested secondary oocytes are then released from the ovary and complete the meiotic division by stimulation of fertilization. Fig.1 shows the stages from the oocyte arrested at the first meiotic prophase (prophase I) via matured egg to fertilization and cleavages. In Fig.1, curved lines indicating MAPK and Cdc2 kinase indicate the activities of mitogen activated protein kinase and Cdc2kinase in the cell cycles of *Xenopus laevis*. The term "oocytes" in the present invention refers to oocytes at the stage from first meiotic prophase to second meiotic metaphase in the cell cycle. The term "eggs" refers to matured oocytes arrested at metaphase II of the meiosis. The previous study of CAT gene expression demonstrated that the gene is more actively expressed when microinjected into the oocytes and dividing embryos, but not into eggs of *Xenopus laevis* (Shiokawa et al., 1994). Nevertheless, the described cell-free protein synthesis system concerning *Xenopus* oocytes reported so far, mainly utilizes the extract obtained from the eggs that have a relatively low capacity for protein synthesis.

In one embodiment of the present invention, translational efficiencies of various cell-free extracts using mRNA encoding Src family kinase (Xyk) of *Xenopus laevis*, as an exogenous mRNA, are disclosed. As a result of investigations with extracts of *Xenopus* oocytes and eggs at various stages in the cell cycle, it has been found that the oocyte extract, in particular, the extract of oocytes just after starting maturation by stimulation, has a remarkably elevated protein synthetic activity, as shown in the following examples.

Thus, in one embodiment of the present invention, there is provided an extract of *Xenopus* oocytes, which extract is prepared from the oocytes at the early stage of maturation entered from the state arrested at the first meiotic prophase in the cell cycle. In this embodiment, maturation of the oocytes may be occurred spontaneously or enforced by artificially administering hormones and the like. The phrase "early stage of maturation" in this embodiment refers to the stage until the completion of first meiotic division after initiation of maturation from the arrested state at first meiotic prophase in the cell cycle, preferably at the initiation stage of the first meiotic division (just before or after the start of first meiotic division) . Whether oocytes are at the initiation stage of the first meiotic division or not, can be decided by the indication of activity of MAPK or Cdc2 kinase in cytoplasm as shown in Fig.1

A cytoplasmic activity from mature eggs that cause complete maturation upon injection into immature oocytes was originally defined by Masui *et al*. as a maturation promoting factor (MPF) (Masui, Y., Differentiation 2001, 69 (1):1-17). It consists of cyclin B and cyclin-dependent protein kinase Cdc2. In the first meiotic prophase-arrested immature oocytes, some part of Cdc2 is stored as an inactive complex, called preMPF, where the catalytic activity of Cdc2 is inhibited by phosphorylations on Thrl4 and Tyr15. Besides the low activities of MPF and CSF are important for maintaining oocytes at the first meiotic prophase. The *Xenopus* MAPK cascade, including Mos, MAPKK, and MAPK is a major component of CSF that causes the metaphase arrest of oocyte in the cell cycle. Microinjections of Mos, or MAPKK mRNA into oocytes, or MAPK protein itself can induce complete MPF activation and meiotic progression in immature oocytes, whereas preventing MAPK activation by MPK1 phosphatase or by MAPKK inhibitor suppresses MPF activation and progesterone-induced oocyte maturation.

Therefore, by any method of regulating the cell cycle described above, for example, treatment of the oocytes arrested at the first meiotic prophase with an egg maturation inducer, or activation of any protein in the MAPK cascade, the oocytes can be entered into the early stage of maturation. It was reported that a morphological marker of meiosis I, germinal vesicle break down (GVBD) can be equally triggered by progesterone, testosterone, a kind of glucocorticoids, and a kind of mineralocorticoids (Morrill, G.A., Bloch, E., J. Steroid Biochem, 1977, 8(2):133-9). Thus, an egg maturation inducer is preferably one or a combination of more than two selected from the group consisting of glucocorticoid, mineralocorticoid, progesterone, testosterone and insulin, more preferably progesterone. The concentration and time of treatment of the egg maturation inducer can be controlled so as to enter the oocytes arrested at the first meiotic prophase into the early stage of maturation, for example, in case of progesterone, the treatment is in a concentration of 5 to 50µM for 30 minutes to 4 hours, preferably about 10µM for 1 to 3 hours.

Preparation of oocyte extract can be performed according to the known method (see Murray A. W., 1991, Cell cycle extracts, Methods Cell Biol 36:581-605). For example, immature stage VI oocytes are amassed by the injection of a hormone into *Xenopus laevis*. Ovaries are isolated from the frog and the stage VI oocytes are obtained by manual and enzymatic treatment such as collagenase. Subsequently, the oocytes are crushed to recover oocyte cytoplasm. To increase the stability of obtained extract and efficiency of protein expression, the oocyte extract can be prepared by supplying with additives such as a cytostatic factor, protease inhibitor and energy substance. After the process to obtain the stage VI oocyte, or simultaneously therewith, the obtained oocytes are preferably treated with an egg maturation inducer such as progesterone. The conditions of progesterone-treatment can be controlled so as to enter the oocytes arrested at the first meiotic prophase into the early stage of maturation, for example, in a concentration of 5 to 50µM for 30 minutes to 4 hours, preferably about 10µM for 1 to 3 hours. The crush of the oocytes may be performed with a usual method such as homogenization and sonication, however, the cytoplasmic fraction can be easily obtained when the cell membrane is disrupted by centrifugation. The obtained cytoplasm fraction is further filtered through the porous membrane to remove cellular debris. The extract can be prepared from any numbers of oocytes, however, a certain number or more of oocytes are preferably treated for easy treatment of crushing or easy addition of stabilizers. In one embodiment of the present invention, the oocyte extract is prepared from at least 100 oocytes.

Thus prepared oocyte extract are to be fresh, for example, within about 8 hours, preferably about 3 hours, for obtaining higher protein synthetic activity, it can be also stored by freezing at -80 to -196°C. To prevent the reduction of protein synthetic activity by freezing, various stabilizers are preferably added to the extract. Alternatively, it is also possible to lyophilize the extract by known methods, for example, after rapid freezing of the oocyte extract by liquid nitrogen, it is dried with a typical lyophilizer.

### (Cell-free protein synthesis kit)

The oocyte extract of the present invention constitutes a cell-free protein synthesis kit with an energy regeneration system and at least one amino acid. The term "energy regeneration system" means a component associating the regeneration of energy source such as ATP and GTP necessary for protein synthesis, and, for example, enzymes relating to ATP regeneration (creatine kinase, pyruvate kinase and the like) and/or substrates thereof (creatine phosphate, phosphoenol pyruvate and the like). Amino acids include at least one amino acid, preferably naturally occurring 20 kinds of amino acids, but it may include other unnatural amino acids. The kit further includes other components such as buffers (for example, HEPES-potassium, and Tris-acetate etc.), various salts, detergents, RNA polymerase (T7, T3 and SP6 RNA polymerases etc.), chaperone proteins (DnaJ, DnaK, GroE, GroEL, GroES, and HSP70 etc.), RNAs (mRNA, tRNA etc.), protease inhibitors, or (ribo)nuclease inhibitors. These components can be simultaneously included two or more. The cell-free protein synthesis kit of the present invention may be a mixture comprising at least the above extract, energy regeneration system, and amino acids upon use, and these components to constitute a kit may be stored separately before use.

In a preferable embodiment of the present invention, the cell-free protein synthesis kit can further include a plasmid DNA for the preparation of exogenous polynucleotide. The term "exogenous polynucleotide" refers to a natural or artificial nucleic acid molecule or nucleic acid construct derived from heterogenous organismus or heterogenous cells. The nucleic acid molecule may be DNA, RNA or analogues thereof, and it may be natural or artificially synthesized. These nucleic acids can be covalently or non-covalently linked to other molecules such as proteins, lipids, chemical compounds, chemical moieties, carriers or magnetic beads.

The exogenous polynucleotide of the present invention is capable of expressing a protein in *Xenopus* oocytes extracts, and can be prepared using expression vectors comprising DNA that encode desirable proteins. When the extract contains a transcriptional activity, for example, endogenous or exogenous RNA polymerase, the expression vectors can be directly added. The expression vector comprises a promoter sequence upstream the protein coding sequence, which starts the transcription. The promoter sequence is not particularly limited to but includes various RNA polymerase promoters to synthesize a single stranded mRNA, such as preferably *Xenopus* RNA polymerase promoter, T7 RNA polymerase promoter, T3 RNA polymerase promoter, and SP6 RNA polymerase promoter. Thus, the phrase "plasmid DNA for the preparation of exogenous polynucleotide" refers to a plasmid DNA comprising an RNA polymerase promoter and polyadenylation signal, wherein a desirable DNA fragment can be cloned.

In a further preferable embodiment of the present invention, mRNA synthesized and modified *in vitro* is used as an exogenous polynucleotide. *In vitro* transcription system is, for example, a transcription reaction system derived from T7 phage and that from *E. coli*. Synthetic reaction of mRNA with this system can be carried out using a commercially available kit such as MEGAscript™ (Ambion) , RiboMAX™ (Promega) and the like. When a eukaryotic mRNA is expressed, it is further preferable to add the cap structure at its 5'-end and poly-A at its 3'-end as a post-transcriptional modification. Most eukaryotic mRNAs have m⁷G(5')ppp(5')G cap structure at its 5'-end, and this cap structure is important for binding with translation initiation factors. It is also known that this structure improves the translational efficiency by stabilizing mRNA. For example, a capped transcript can be easily synthesized by adding a cap analog (7-methyl-guanosine) into *in vitro* transcription system. On the other hand, poly A addition at the 3'-end is catalyzed by, for example, poly A polymerase. Poly A addition prevents the cleavage from the 3' end of mRNA by exonucleases and can stabilize mRNA.

### (Method of producing proteins by cell-free protein synthesis system)

The protein synthesis in cell-free protein synthesis system using a *Xenopus* oocyte extract is essentially carried out by adding the mRNA to the cell-free extract having the aforementioned protein synthetic activity. For example, mRNA encoding a target protein, and its constituents such as amino acids and energy source (ATP, GTP and the like) are added to the reaction mixture containing the oocyte extract, and the reaction mixture is incubated usually at 20 to 40°C, preferably at 23 to 30°C, to synthesize the target protein. The cell-free protein synthesis system of the present invention can be employed a batch method, flow method, and any other techniques previously known, for example, ultrafiltration method, dialysis method, column chromatography method using a resin on which templates for translation are immobilized (refer to Spirin, AS. et al., Meth. In Enzymol. Volume 217, pp. 123-142, 1993), and the like. As for the above dialysis method, there is disclosed a method described in Kigawa, T. *et al*. FEBS Letters 1999, Vol. 442, pp. 15-19, and Japanese Patent Kokai publication, JP-A-2000-175695, which method is carried out by introducing the reaction mixture inside a dialysis membrane having a fractional molecular weight of 10,000 or more, preferably 50,000 or more, and dialyzed against the external dialysate (comprising amino acids, energy sources and the like) of 5 to 10-fold volume compared to the reaction mixture. The dialysis is performed usually at 20 to 40°C, preferably at 23 to 30°C, with agitation, and when the reaction rate decreases, the external dialysate is exchanged with fresh one.

Further, the above cell-free protein synthesis system has a translational activity to synthesize protein, and a post-translational modification activity such as glycosylation and phosphorylation. Therefore, by adding the mRNA to the cell-free extract, the protein is synthesized from the mRNA and then further modified to synthesize a glycoprotein or a phosphorylated protein.

When the glycoprotein or phosphorylated protein is synthesized as described above, the cell extract can be prepared by adding a cytostatic factor, protease inhibitor, ribonuclease inhibitor, and energy substance to the cell extract. As an example, the oocyte extract can be prepared in a final concentration of 10 µg/ml each cytochalasin B, leupeptin, pepstatin, and chymostatin, 1 mM spermidine, 7.5 mM creatine phosphate, 1 mM ATP, and 1 mM MgCl₂, to provide for translation reaction. In addition, amino acid mixture is preferably added to the cell extract. The added amino acids are, for example, about 25 µM respectively in a final concentration, which may be any of natural or unnatural.

The amount of mRNA added to the cell-free protein synthesis system of the present invention is, for example, 10 to 100 µg/ml in a final concentration of the oocyte extract. Alternatively, when the transcription/translation coupled reaction is carried out in the cell-free protein synthesis system, DNA constructed as the expression vector is directly added. In case of the transcription activity in the oocyte extract is low, T7, T3, or SP6 RNA polymerase is added with ribonucleotide triphosphates (ATP, GTP, CTP, UTP) and the like.

The protein synthesized by the production method of the present invention, may be any protein derived from eukaryotic or prolaryotic, and glycosilated or phosphorylated as a post-translational modification. The protein forms a characteristic three-dimensional or four-dimensional structure to exert the function of the protein. Thus, the synthesized protein can be used for its structural and functional research and also for drug screening using an interaction with other protein or low molecular weight compound.

### [Examples]

The present invention is explained in more detail by the following examples, which describe the detailed analysis of the expression of mRNA encoding *Xenopus* Src family kinase (Xyk) in extracts of *Xenopus* oocytes and eggs, or in oocytes microinjected with the mRNA. These examples are intended to describe the invention more clearly and not to limit the invention in any respect. In the examples, preparation of *Xenopus* oocytes and eggs, preparation of egg and oocyte extract, mRNA preparation, microinjection, translation reaction in cell-free protein synthesis system, immunoprecipitation, immunoblotting, and protein kinase assays are carried out according to the following procedures.

### [Preparation of Xenopus oocytes and eggs]

Wild type African clawed frogs *(Xenopus Laevis)* were purchased from Hamamatsu Seibutsu Kyozai (Hamamatsu, Japan). Frogs were maintained in polypropylene tanks filled with deionized water (2L per animal) at temperature of 21-23°C with a twice-a-week feeding. Frogs were primed with pregnant mare serum gonadotropin (50 U/animal, Biogenesis) 5-7 days before the experiments.

To obtain oocytes, frogs were anesthetized on ice, then ovaries were surgically removed and placed into OR-2 solution containing 82.5 mM NaCl, 2.5 mM KCl, 1 mM CaCl₂, 1 mM MgCl₂, 1 mM Na₂HPO₄, 5 mM HEPES, pH 7.8. Ovaries were manually dissected into clumps of 50-100 oocytes and extensively washed with OR-2 solution. The clumps of oocytes were treated with 0.5 mg/ml collagenase (Wako, 280 U/mg) in OR-2 at 23°C for 3 hours by shaking at 60 revs/min. Liberated oocytes were washed with OR-2 solution and left for stabilization over 4 hours. Undamaged stage VI oocytes were manually selected. Maturation was induced by the addition of progesterone (Sigma) to a final concentration of 10µM. Meiotic transition was monitored by occurrence of germinal vesicle breakdown (GVBD) judged by appearance of a white spot on the animal hemisphere of oocytes.

Eggs were obtained after inducing ovulation by the injection of human chorionic gonadotropin (500 U/animal, Teikokuzoki, Japan). In 8-12 hours after injection, eggs were squeezed from the frogs into a plastic dish filled with MMR solution (100 mM NaCl, 2 mM KCl, 1 mM MgCl₂, 2 mM CaCl₂, 0.1 mM EDTA, 5 mM HEPES), dejellied by treatment with 2% cysteine (Sigma) in MMR, and washed extensively with the extract buffer, containing 100 mM KCl, 0.1 mM CaCl₂, 1 mM MgCl₂, 5 mM EGTA, 50 mM sucrose, and 10 mM potassium HEPES, pH 7.7.

### [Egg and oocyte extract preparation]

Cytostatic factor-arrested extracts of *Xenopus* eggs were prepared from the unfertilized eggs essentially according to the method of Murray et al.,(supra). The eggs were transferred to the centrifuge tubes containing extract buffer plus 100 µg/ml cytochalasin B (Sigma) and 10 µg/ml each leupeptin, pepstatin, and chymostatin. Tubes were centrifuged for 30 sec at 1,000 rpm, then for 30 sec at 1,500 rpm at 4°C. All buffer was removed from the top of the tubes and eggs were crushed by a centrifugation for 15 minutes at 12,000 rpm. The cytoplasmic layer between lipid cap and sedimented yolk was collected and subjected to the second clarifying centrifugation under the same conditions. Cytochalasin B, protease inhibitors, and energy mix (1/20 volume of 150 mM creatine phosphate, 20 mM ATP, 20 mM MgCl₂) were added to the extracts that were kept on ice until use. All experiments with the extracts were performed at 23°C within 3 hours after preparation. Interphase released egg extracts were obtained by the addition of calcium (final concentration 0.5 mM) to the cytostatic factor-arrested metaphase extracts. Metaphase/interphase transition in the extracts usually took about 30 minutes and was confirmed by the change in the nuclear morphology of demembraned sperm nuclei added to extracts. Nuclear morphology was observed and scored by fluorescent microscopy after removing 1µl of extract and adding 4 µl of stain solution (1 µg/ml Hoechst 33342 (sigma) in MMR buffer containing 10% formaldehyde and 50% glycerol). Oocyte extracts were prepared from the selected stage VI oocytes washed in the extract buffer by the same procedure described above for egg extracts.

### [mRNA preparation]

The full length *Xenopus* Src kinase (Xyk) DNA (refer to GenBank Accession No. M23422) containing two amino acid substitutions at positions (Y527F and R121A) was subcloned into pBluescript II vector (Toyobo) with the upstream T7 promoter. *In vitro* RNA transcription coupled with capping reaction by 7-methyl-guanosine at the 5' end was performed from SpeI-linearized plasmid using mMESSAGEmMASHINE T7 high yield capped RNA transcription KIT (Ambion), according to the manufacturer's manual. Purification of the synthesized mRNA was carried out with RNeasy purification KIT (Qiagen). Post-transcriptional polyadenylation of synthetic mRNA at the 3' end with *E*. *coli* poly(A) polymerase was done according to the method of Wormington et al. (see Wormington, M., 1991, Methods Cell Biol 36:167-183). Polyadenylation reaction was performed at 37°C for 5 minutes in the reaction mixture containing 50 mM Tris-HCl, pH 8.0, 250 mM NaCl, 10 mM MgCl₂, 1 mM MnCl₂, 2 mM DTT, 50 units of RNAse inhibitor, 50 µg/ml bovine serum albumin, 50 µM ATP, 5 µg synthetic RNA in a final volume of 50 µl. After purification with RNeasy purification KIT, RNA was dissolved in RNase free water at 1 mg/ml which solution was used for microinjections into eggs and oocytes or direct additions into the extracts. RNA quality was controlled by denaturing agarose/formaldehyde electrophoresis, performed essentially as described Sambrook et al. (Sambrook, J. et al., Molecular Cloning).

### [Microinjection]

Quantitative injection of mRNA into stage VI *Xenopus* oocytes was made under microscopic observation with a pulse-directed injector system (Drummond, Nanoject). RNA solution was loaded into the oil-filled glass microcapillary needles with a tip diameter of 10-30 µm. Fifty ng of RNA dissolved in RNase-free water was injected into an oocyte in a total volume of 50 nl.

### [Translation reaction in cell-free protein synthesis system]

The freshly prepared egg or oocyte extracts supplemented with 10 µg/ml of cytochalasin B, leupeptin, pepstatin, and chymostatin, as well as the energy mix (1/20 volume of 150 mM creatine phosphate, 20 mM ATP, 20mM MgCl₂) were used. The extracts were incubated with in vitro synthesized *Xenopus* Src RNA or Luciferase Control RNA (Promega) as a contrast at final concentrations of 50 ng/µl or 20 ng/µl, correspondingly, in the presence of 1 mM spermidine and 1 U/µl ribonuclease inhibitor RNasin (Promega). Incubation time was 90-120 minutes at 21-23°C. The final volume of a reaction was 20 µl with the extract dilution less than 20%. The reactions were stopped by freezing in liquid nitrogen.

### [Analysis by Immunoprecipitation]

Frozen eggs, or oocytes, or aliquots of the extracts were ten-fold diluted with a homogenization buffer (20 mM Tris-HCl, pH 7.5, 1% Triton X-100, 1 mM EDTA, 1 mM EGTA, 10 mM β-mercaptoethanol, 1 mM sodium vanadate, 10 µg/ml leupeptin, 20 µM APMSF), then sonicated for 2 minutes on ice with a TOMY UD-201 ultrasonic disrupter (Tomy Seiko, Tokyo). Samples were centrifuged for 10 minutes at 15,000 rpm, then 20-µl aliquots of supernatants were incubated with 5 µl of anti-Src family kinase mAb327 antibody (Ab-1, Oncogene) for 2 hours at 4°C. To collect immune complexes, protein A-Sepharose was added to the samples at a final concentration of 10% and the mixture was left for 1 hour. Nonspecifically bound proteins were washed with the buffer containing 50 mM Tris-HCl, pH 7.5, 150 mM NaCl, 1% Triton-X-100, 1% sodium deoxycholate, 0.1% SDS. For the analysis of expression level, samples were treated with SDS-PAGE sample buffer and subjected to immunoblotting analysis as described below. To confirm the expression of functionally active *Xenopus* Src, protein kinase assay of immunoprecipitated samples was performed.

### [Analysis by Immunoblotting]

Immunoprecipitated samples or 20-µl aliquots of ten-fold diluted extracts were mixed with a concentrated SDS-PAGE sample buffer. Proteins were separated by the electrophoresis on 10% polyacrylamide gels and transferred to PVDF membranes using a semidry blotting device (Bio-Rad). Membranes were blocked with T-TBS buffer (20 mM Tris-HCl, pH 7.5, 150 mM NaCl, 0.05% Tween 20) containing 3 mg/ml bovine serum albumin and incubated for 2 hours with 100-fold diluted anti-pep Y serum (Fukami et al., J. Biol. Chem. 1993, 268:1132-1140). After washing, the membranes were treated with anti-mouse IgG rabbit polyclonal antibody (Cappel) at a 500-fold dilution, then with a 1000-fold diluted alkaline phosphatase-conjugated goat polyclonal antibody against rabbit IgG (Santa Cruz). Membranes were thoroughly washed with T-TBS buffer and incubated in the developing buffer (100 mM Tris-HCl, pH 9.5, 5 mM MgCl₂, 100 mM NaCl, 50 µg/ml 5-bromo-4-chloro-3-indolyl phosphate p-toluidine salt, and 150 µg/ml nitro blue tetrazolium) to visualize the immune complexes.

### [Protein kinase assays]

Total tyrosine kinase activity of the immunoprecipitated sample was measured with poly (Gly, Try) 4:1 (Sigma), as a protein substrate, and specific activity of immunoprecipitated *Xenopus* Src family kinase was estimated with a synthetic cdc2 peptide, corresponding to residues 7-26 of the fission yeast cdc2 gene product (Fukami et al., supra). The samples of extracts were five-fold diluted with a kinase dilution buffer (80 mM β-glycerophosphate, pH 7.5, 20 mM EGTA, 15 mM MgCl₂, 1 mM DTT, 0.1 mM NaF, 1mM Na₃VO₄, 0.2 mM APMSF, 10 µg/ml leupeptin, 10 µg/ml aprotinin). Prior to the kinase assay, immunoprecipitated samples were washed with the kinase dilution buffer. The reaction mixture of protein kinase assay (20 µl) contained 50 mM Tris-HCl, pH 7.5, 5 mM MgCl₂, 1 mM dithiothreitol, 0.5 mg/ml poly(Gly, Tyr) or 1 mg/ml cdc2 peptide, 2 µM [γ-³²P]ATP(1 µCi), and 5 µl of diluted or immunoprecipitatetd extracts. Samples were incubated for 10 minutes at 30°C, then the reaction was terminated by the addition of concentrated SDS-PAGE sample buffer. After electrophoresis, the radioactive bands of phosphorylated proteins were visualized and quantified by BAS2000 image analyzer (FUJI Film). Incidentally, the expression level measured by the immunoblotting method, or the kinase activity measured by the protein kinase assay was quantified by using a software (ImageGauge V.3.45) that calibrates the target band intensity as an absorbance, and shown as arbitrary units (a.u.).

### [Comparative Example 1] Comparison of expression levels of Xyk in Xenopus egg extracts arrested at the Metaphase and Interphase.

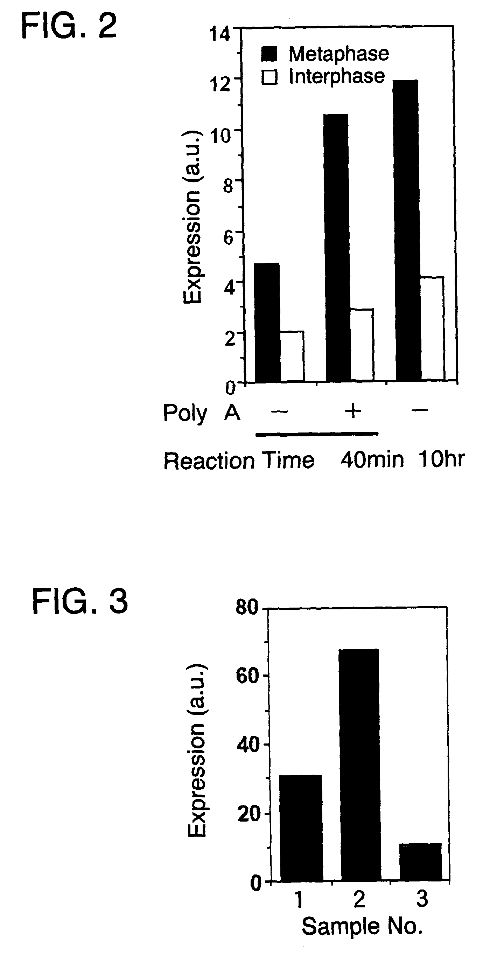

Until now, the reports concerning the expression of *Xenopus* Src kinase in a cell-free extract have not been submitted. Therefore, we compared the expression using mRNA prepared by the above method in the metaphase arrested and interphase extracts of eggs. The result was shown in Fig.2. Fig.2 shows expression levels of Xyk using the mRNA with (+) or without (-) poly A in the extracts prepared from metaphase or interphase of *Xenopus* egg measured by immunoblotting of immunoprecipitated samples with Xyk specific antibody. As shown in Fig.2, it was found that the expression level of Xyk in the extracts released to interphase by the addition of calcium was 2 to 3 fold lower than that in the metaphase extracts in both reaction times 40 minutes and 10 hours irrespective of poly A addition or not.

### [Example 1] Comparison of expression levels of Xyk in microinjected oocytes, and extracts of oocytes or eggs.

Next, the expression levels in microinjected *Xenopus* oocytes, an oocyte extract, and an egg extract using mRNA prepared by the above method were shown in Fig. 3. Sample No.1 in Fig.3 shows the expression level in the oocytes microinjected with the mRNA followed by 24 hour incubation, which oocytes are arrested at the first meiotic prophase. Sample No.2 shows the expression level in the oocyte extract with the mRNA followed by 90 minute incubation, which oocyte is also arrested at the first meiotic prophase. Sample No.3 shows the expression level in the metaphase egg extract with mRNA followed by 90 minute incubation. As shown in Fig.3, not only the expression level of Xyk in the extract of oocytes arrested at the first meiotic prophase (sample No.2) was much higher than that in the metaphase egg extracts (sample No.3), but also it significantly exceeded the level of expression in the mRNA microinjected oocytes (sample No.1).

### [Example 2] Analysis of correlation between the oocyte maturation by progesterone-treatment and expression levels of Xyk.

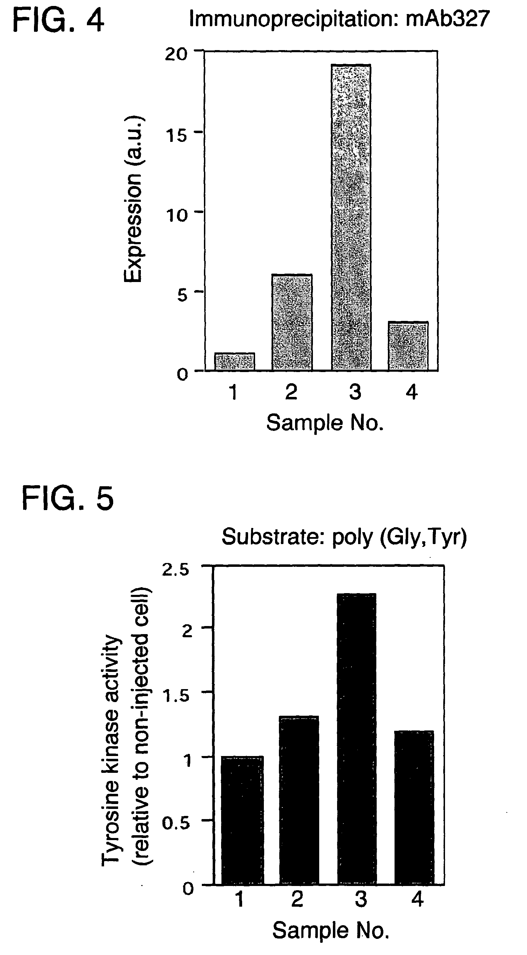

Next, we investigated the effect of progesterone treatment on the expression of Xyk in the oocytes or in the oocyte extracts. After the oocytes microinjected with Xyk mRNA were treated with progesterone in various conditions, the expression levels of Xyk were measured by immunoblotting (Fig.4) and protein kinase assay (Fig.5). In each figure, sample No.1 shows the expression level of Xyk after 4 hour incubation at 23°C without mRNA microinjection. The minor band derived from endogenous Srk kinase was detected (data not shown). Sample No.2 shows the expression level in the oocyte microinjected with Xyk mRNA after 4 hour incubation at 23°C in the absence of progesterone. Sample No.3 shows the expression level in the oocyte microinjected with Xyk mRNA after 4 hour incubation at 23°C in the presence of 10 µM progesterone. Sample No.4 shows the expression level in the microinjected oocyte with Xyk mRNA, which oocytes are pre-treated with 10 µM progesterone for 4 hours, and then further incubated at 23°C for 4 hours in the absence of progesterone. These results indicated that the progesterone treatment after mRNA microinjection increased the Xyk expression about two times, but the pre-treatment of the oocytes with progesterone reversely decreased the expression level. The reason why the expression level was reduced is speculated to be due to the cell cycle progression of the oocytes to the state of eggs (see Fig.1), which is consistent to the results of Example 1 (Fig.3).

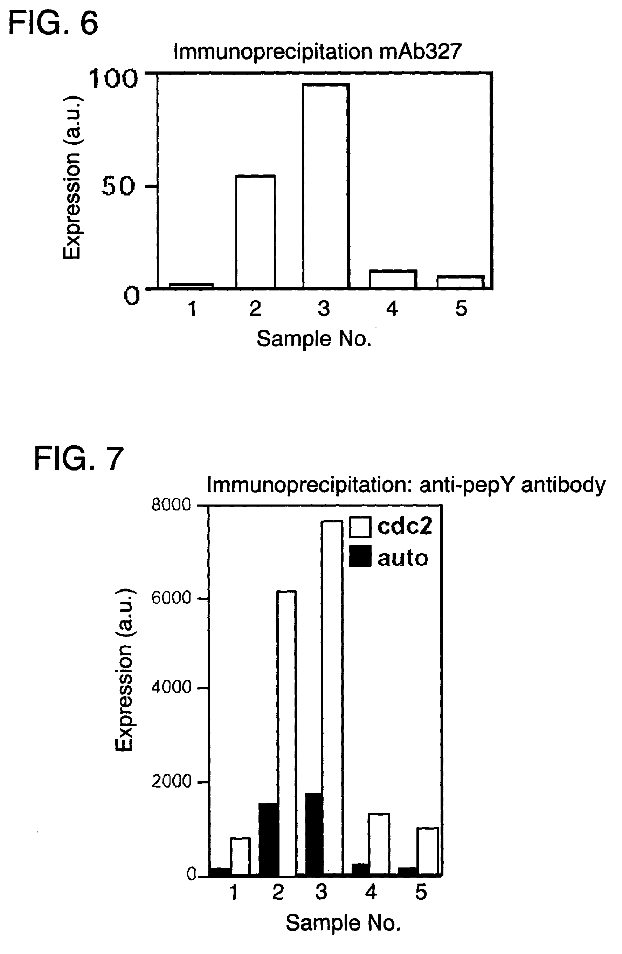

The expression levels of Xyk in the oocyte extract added with the mRNA, which oocytes were treated with progesterone in various conditions, were measured by immunoblotting (Fig.6) and protein kinase assay (Fig.7). In each figure, sample No.1 shows the expression level of Xyk without addition of the mRNA. Sample No.2 shows the expression level in the extract of oocytes without progesterone treatment. Sample Nos.3 to 5 show the expression levels in the extracts of oocytes treated with 10µM progesterone for 2, 4, and 8 hours, respectively. These results indicated that the expression of Xyk was increased only in the extract prepared from oocytes treated with progesterone for 2 hours, but was decreased in the extracts prepared from oocytes treated with progesterone for 4 and 8 hours respectively. The reason why the expression level was reduced is speculated to be due to the cell cycle progression of the oocytes to the state of eggs. In addition, the increase of the expression level was about 1.5 times, which was less than the result in Fig. 4 (about three time increase compared to the expression level in the oocytes without progesterone treatment). This suggests that the progesterone treatment for less than 2 hours makes it possible to enhance the expression level. From the above results, both in the oocytes and extracts, short time (2 hour) progesterone treatment led to the stimulation of Xyk production, whereas longer treatment (4-8 hours) or pretreatment suppressed the synthesis much below the level of the untreated control. Also, the results presented in Figs. 4 to 7 demonstrate that the synthesized Xyk is functionally active (has a tyrosine kinase activity).

### [Example 3] Determination of expression level in oocyte extract

To estimate the Xyk expression level in *Xenopus* oocyte extracts, the reaction mixture for 90 minutes after addition of the mRNA was electrophoresed, and calculated the protein amount by CBB staining of the band. The estimation gave the value of 7.8 µg/ml, in comparison with bovine serum albumin (BSA), as a standard.

### [Reference Example 1] Kinetics of Xyk synthetic rate in the extracts

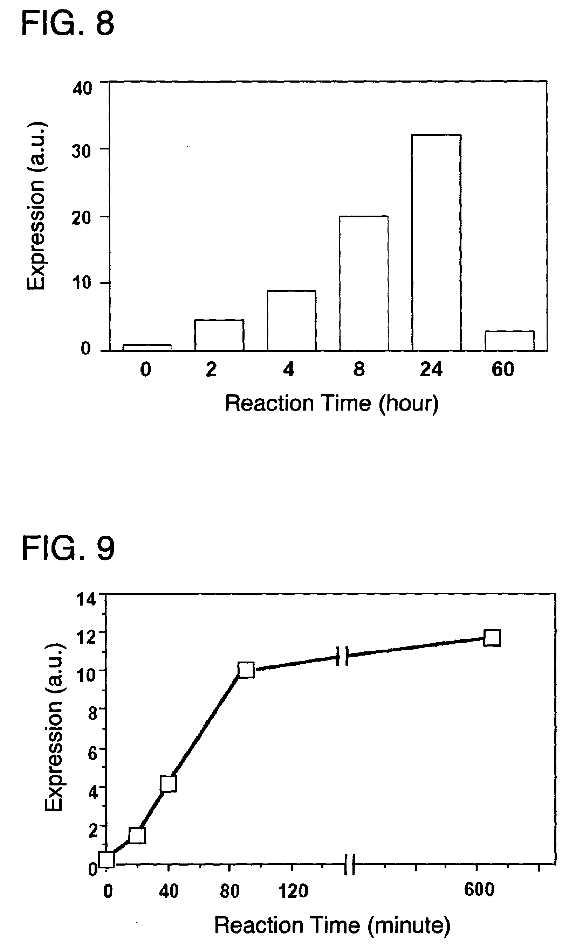

To further optimize the conditions of protein production in the extracts, the kinetics of Xyk synthesis in the microinjected oocytes and egg extracts were determined and shown in Figs. 8 and 9, respectively. In accordance with the previous reports, translation in the cell-free extracts (saturated at 90 minute incubation, see Fig. 9) was much faster than that of microinjected oocytes (increasing until 24 hours, see Fig.8).

### Industrial Applicability

The *Xenopus* oocyte extract of the present invention, shows a significant higher protein synthetic activity than that in the egg extract and microinjected oocytes. Thus, the extract can be used for various uses of translation of desired protein, especially an eukaryotic protein, and post-translational modification such as glycosylation, phospholylation , cleavage of signal sequence, localization, and formation of protein higher structure. The present invention provides a novel cell-free protein synthesis system that can be used to alternatively express the proteins, in particular, an eukaryotic protein, which can not be successively expressed in the known cell-free system.

## Claims

1. An extract of *Xenopus* oocytes, which extract is prepared from oocytes at the early stage of maturation entered from the state arrested at the first meiotic prophase in the cell cycle.

2. The extract of claim 1, wherein said oocytes at the early stage of maturation are obtained by treating stage VI immature oocytes with an egg maturation inducer.

3. The extract of claim 1, wherein said oocytes at the early stage of maturation are obtained by incubating stage VI immature oocytes in the presence of 5 to 50 µM progesterone for 0.5 to 4 hours.

4. The extract of claim 1, wherein said early stage of maturation is the initiation stage of the first meiosis.

5. The extract of claim 1 prepared from at least one hundred oocytes.

6. The extract of claim 1, further being supplemented with at least one selected from the group consisting of a cytostatic factor, protease inhibitor, and energy substance.

7. A kit for use in a cell-free protein synthesis comprising:
an extract prepared from *Xenopus* oocytes at the early stage of maturation entered from the state arrested at the first meiotic prophase in the cell cycle,
an energy regenerating system, and
at least one amino acid.

8. The kit for use in a cell-free protein synthesis of claim 7, further comprising a plasmid DNA for the preparation of exogenous polynucleotide.

9. A method of producing a protein in a cell-free protein synthesis system comprising the steps of:
preparing a polynucleotide coding for a protein, and
expressing said polynucleotide in an extract prepared from *Xenopus* oocytes at the early stage of maturation entered from the state arrested at the first meiotic prophase in the cell cycle.

10. The method of claim 9, wherein said oocytes are oocytes at entry to an early stage of maturation by treating oocytes arrested at the first meiotic prophase with an egg maturation inducer.

11. The method of claim 10, wherein said egg maturation inducer is at least one selected from the group consisting of glucocorticoid, mineralcorticoid, progesterone, testosterone, and insulin.

12. The method of claim 9, wherein said polynucleotide is an exogenous mRNA that is synthesized and modified *in vitro.*
